# EUROPÄISCHE PATENTSCHRIFT

(11) **EP 4 407 632 B1**
(45) Veröffentlichungstag und Bekanntmachung des Hinweises auf die Patenterteilung: **31.12.2025**
(21) Anmeldenummer: 23153704.4
(22) Anmeldetag: 27.01.2023
(51) Int. Cl.: G16H 50/30, G16H 20/40, G16H 40/63, G16H 10/60, A61B 5/00, G01G 19/44

(54) **VERFAHREN ZUM BESTIMMEN EINES PATIENTENGEWICHTS EINES AUF EIN HILFSMITTEL ANGEWIESENEN PATIENTEN UND SYSTEM**
METHOD FOR DETERMINING A PATIENT WEIGHT OF A PATIENT IN NEED OF AN ADJUVANT AND SYSTEM
PROCÉDÉ DE DÉTERMINATION D'UN POIDS DE PATIENT D'UN PATIENT EN AYANT BESOIN D'UN DISPOSITIF AUXILIAIRE ET SYSTÈME

(43) Veröffentlichungstag der Anmeldung: 31.07.2024
(73) Patentinhaber: MedVision AG, 59423 Unna (DE)
(72) Erfinder: KOPECKY, Jens, 40627 Düsseldorf (DE)
(74) Vertreter: Angerhausen, Christoph

(56) Entgegenhaltungen:
- JP-A- 2021 163 318
- US-A1- 2013 274 644

## Beschreibung

Die Erfindung betrifft ein computerimplementiertes Verfahren zum Bestimmen eines Patientengewichts eines auf ein Hilfsmittel angewiesenen Patienten, und ein entsprechendes System.

Personen oder Patienten mit einem Nierenversagen, d.h. bei nicht-Funktion oder nicht ausreichender Funktion einer oder der Niere eines Patienten, sind auf eine Dialysebehandlung angewiesen, die Blut des Patienten reinigt, z.B. von Elektrolyten oder Abfallprodukten, und damit eine Nierenersatzfunktion darstellt.

Aufgrund nicht oder nur schwach funktionierender Nieren können auf Dialyse angewiesene Personen oder Patienten Flüssigkeit, z.B. Wasser, nicht oder kaum abscheiden, so dass sich Flüssigkeit und/oder Wasser im Körper ansammelt, wodurch diese Personen oder Patienten an Gewicht zunehmen. Die Gewichtszunahme kann dabei im Wesentlichen der Flüssigkeitsaufnahme der Person bzw. des Patienten entsprechen.

Da derartige Flüssigkeitsansammlungen gesundheitsschädlich sein können, bzw. in zu hoher Menge gesundheitsschädlich sind, muss die sich in dem Körper ansammelnde Flüssigkeit entfernt werden. Eine derartige Flüssigkeitsentfernung kann beispielsweise durch Ultrafiltration (UF) während einer Dialysebehandlung, beispielsweise einer Hämodialyse, erfolgen. Dabei wird dem Patienten entnommenem Blut Wasser entzogen, bevor das Blut dem Patienten wieder zugeführt wird.

Um die Menge an zu entfernendem Wasser zu bestimmen, wird dabei üblicherweise das Gewicht des Patienten mit einem Sollgewicht, z.B. einem "Trockengewicht" des Patienten, verglichen. Die Menge an bei der Dialyse zu entfernendem Wasser wird dabei in vielen Fällen aus der Differenz des Körpergewichts des Patienten abzüglich des Sollgewichts, bzw. abzüglich des Trockengewichts, bestimmt.

Die bei einer Dialysebehandlung zu entfernende und/oder entfernte Menge an Flüssigkeit und/oder Wasser wird häufig als UF-Volumen (Ultrafiltration-Volumen) bezeichnet. Ein kg Wasser hat unter üblichen Bedingungen im Wesentlichen ein Volumen von einem Liter, so dass das Gewicht der zu entfernenden und/oder entfernten Flüssigkeit und/oder des Wassers in ein Volumen bzw. das UF-Volumen umrechenbar ist und/oder diesem entspricht.

Eine UF-Rate (Ultrafiltration-Rate) ist häufig als die Geschwindigkeit, mit der die Menge bzw. das UF-Volumen entfernt wird, definiert. Die UF-Rate kann die Einheit [m³/s], oder eine Umrechnung, z.B. [L/h] haben. Bei zu hoher UF-Rate können Nebenwirkungen oder Schädigungen des Patienten auftreten, und/oder kardiovaskuläre Risiken erhöht oder Krankenhausaufenthalte aufgrund von Herz-Kreislauf-Erkrankungen wahrscheinlicher werden. Die Grenzwerte zu hoher UF-Rate sind dabei üblicherweise von dem Patientengewicht abhängig. Eine spezifische UF-Rate kann deshalb als das Verhältnis aus UF-Rate zu Körpergewicht bzw. als UF-Rate zu Patientengewicht definiert sein, wobei die Risiken mit steigender spezifischer UF-Rate (bzw. für einen gegebenen Patienten entsprechender mit zunehmender UF-Rate) bei Überschreiten des Grenzwerts zunehmen.

In vielen Fällen müssen sich auf Dialyse angewiesene Patienten mehrmals pro Woche, beispielsweise dreimal pro Woche oder häufiger, einer Dialysebehandlung unterziehen, um Blut zu reinigen und/oder Wasser zu entfernen.

Auf eine Dialyse angewiesene Patienten müssen sich deshalb regelmäßig wiegen oder gewogen werden, um das Körpergewicht zu bestimmen. Üblicherweise werden Patienten mindestens vor einer Dialysebehandlung gewogen, um das Patientengewicht und/oder die Abweichung vom Sollgewicht zu bestimmen. Sehr häufig wiegen sich Dialysepatienten aber auch häufiger (oder werden häufiger gewogen), um die Flüssigkeitsmenge im Körper zu bestimmen.

Das Verfahren und/oder das System ist aber auch allgemein zur Erfassung eines Patientengewichts auf ein Hilfsmittel angewiesener Patienten geeignet.

Auf ein Hilfsmittel angewiesene Patienten, beispielsweise Rollstuhlfahrer, Krückenträger, Prothesenträger oder sonstigen auf Gehhilfen angewiesene Patienten, können sich häufig nicht selbst wiegen, sondern müssen gewogen werden, z.B. durch Ärzte, Pfleger oder Angehörige, bzw. sind auf Hilfe angewiesen. Damit ist die Bestimmung des Körpergewichts bzw. Patientengewichts von auf ein Hilfsmittel angewiesenen Patienten nicht immer unkompliziert oder einfach.

Bei bekannten Verfahren wird der Patient mit seinem Hilfsmittel gewogen. Anschließend wird von dem ermittelten Gewicht ein konstantes Gewicht des Hilfsmittels abgezogen. Diese Verfahren können damit nur dann das Patientengewicht korrekt bestimmen, wenn das Gewicht des Hilfsmittels z.B. aus Herstellerangaben oder dergleichen bereits bekannt ist. Zudem kann nicht nur das Gewicht des Patienten, sondern auch das Gewicht des Hilfsmittels variieren, so dass das mit dem bekannten Verfahren ermittelte Gewicht fehlerhaft sein kann. Beispielsweise können Rollstühle Taschen haben, die bei verschiedenen Messungen verschieden gefüllt sein können, so dass das Gewicht des Hilfsmittels bzw. des Rollstuhls nicht bei den verschiedenen Messungen konstant bzw. gleich ist. Die entsprechende Bestimmung des Patientengewichts ist damit fehleranfällig und vergleichsweise aufwendig, da nicht vergessen werden darf, die Rollstuhltaschen vollständig zu leeren.

In einigen Kliniken, Praxen oder dergleichen ist das Wiegen bzw. Bestimmen des Patientengewichts aufgrund Personalmangels oder wirtschaftlicher Erwägungen den Angehörigen des Patienten überlassen, die mit Feinheiten der Messung oder Bedienung der Waage oder dergleichen nicht vertraut sind oder sein können, so dass die Messung mit einer vergleichsweise großen Unsicherheit belastet ist, oder zu hoch bestimmt wird.

Ein Verfahren zur Bestimmung eines Patientengewichts, welches die Gewichtsinformationen von per Gesicht identifiziertem Patient und Rollstuhl in einer Datenbank abspeichert, ist aus der JP 2021 163318 A bekannt. Ein Dialysesystem ist aus der US 2013/0274644 A1 bekannt.

Es ist deshalb Aufgabe der vorliegenden Erfindung, ein Verfahren und ein System bereitzustellen, das einfach zu bedienen ist und ein Patientengewicht eines auf ein Hilfsmittel angewiesenen Patienten sicher erfassen kann. Das Verfahren und System kann zudem die erfassten Gewichtswerte auf Plausibilität prüfen, so dass fehlerhafte Eingaben oder Messungen erkannt und/oder nicht berücksichtigt werden können.

Diese Aufgabe wird durch ein Verfahren mit den Merkmalen des Anspruchs 1 und ein System mit den Merkmalen des Anspruchs 12 gelöst. Die abhängigen Ansprüche betreffen jeweils vorteilhafte Ausführungsformen.

Ein erster Aspekt der Erfindung betrifft ein Computerimplementiertes Verfahren zum Bestimmen eines Patientengewichts eines auf ein Hilfsmittel angewiesenen Patienten, mit den folgenden Schritten:
a) Erfassen einer eindeutigen ID des Patienten;
b) Erfassen eines ersten Gewichts durch Wiegen des mit einem Hilfsmittel versehenen Patienten, so dass das erste Gewicht der Summe aus dem Gewicht des Patienten und dem Gewicht des Hilfsmittels entspricht;
c) Erstellen einer mit der eindeutigen ID des Patienten verknüpften Gewichtsinformation, und Zuordnen des ersten Gewichts zu der Gewichtsinformation;
d) Erfassen eines zweiten Gewichts durch Wiegen des Hilfsmittels;
e) Zuordnen des zweiten Gewichts zu der Gewichtsinformation; und
f) Bestimmen des Patientengewichts des Patienten durch Subtraktion des zweiten Gewichts von dem ersten Gewicht.

Erfindungsgemäß umfasst das computerimplementierte Verfahren ein Prüfen, ob die Gewichtsinformation eine vollständige Gewichtsinformation ist, wobei das Prüfen umfasst:
- Prüfen, ob das zweite Gewicht erfasst worden ist; und/oder
- Prüfen, ob das erste Gewicht in einem zeitlichen Zusammenhang mit dem zweiten Gewicht steht; und/oder
- Prüfen, ob das erste Gewicht in einem logischen Zusammenhang mit dem zweiten Gewicht steht, wobei bevorzugt ein logischer Zusammenhang vorliegt; und/oder
- Prüfen, ob das zweite Gewicht geringer als das erste Gewicht ist, und/oder Prüfen, ob das zweite Gewicht größer als 0,5 kg und/oder kleiner als 200 kg ist,
wobei, wenn das Prüfen erfolgreich war, die Gewichtsinformation als vollständige Gewichtsinformation markiert wird, und wenn das Prüfen unerfolgreich war, die Gewichtsinformation als unvollständige Gewichtsinformation markiert wird, wobei das Prüfen, ob die Gewichtsinformation eine vollständige Gewichtsinformation ist, vor dem Bestimmen des Patientengewichts durchgeführt wird und das Patientengewicht nur dann bestimmt wird, wenn die Gewichtsinformation als vollständige Gewichtsinformation markiert ist, und/oder wobei, wenn die Gewichtsinformation als unvollständige Gewichtsinformation markiert ist, einer, mehrere oder alle der Schritte a) - e) des Verfahrens wiederholt werden, bevorzugt mindestens die Schritte b) und c) wiederholt werden.

Das Prüfen, ob die Gewichtsinformation eine vollständige Gewichtsinformation ist, kann einer Plausibilitätsprüfung der erfassten ersten und/oder zweiten Gewichte dienen.

Damit kann sichergestellt sein oder werden, dass das Patientengewicht nur dann bestimmt ist oder wird, wenn die erfassten Gewichte bzw. Messwerte sinnvoll und/oder plausibel sind. Es kann vermieden werden, dass unsinnige oder offensichtlich fehlerbehaftete Werte als Patientengewicht bestimmt, gespeichert oder hinterlegt, und/oder einem Patienten zugeordnet werden. Das Prüfen, ob die Gewichtsinformation eine vollständige Gewichtsinformation ist, kann einer Plausibilitätsprüfung der erfassten Gewichte und/oder Messungen dienen. Insbesondere kann das System und/oder das Verfahren durch das entsprechende Prüfen selbstständig erkennen, ob fehlerhafte Messungen oder Werte vorliegen, und/oder ob diese sinnvoll sind.

Ein zeitlicher Zusammenhang kann vorliegen, wenn das Erfassen des ersten Gewichts und das Erfassen des zweiten Gewichts innerhalb eines vorgegebenen Zeitintervalls erfolgt. Es kann alternativ oder zusätzlich vorgesehen sein, dass das vorgegebenen Zeitintervall patientenabhängig ist und/oder von der eindeutigen ID abhängt, und/oder mit der eindeutigen ID verknüpft oder dieser zugeordnet ist. In einigen Ausführungsformen kann das vorgegebene Zeitintervall 24 h betragen.

Ein logischer Zusammenhang kann vorliegen, wenn das Erfassen des ersten Gewichts und das Erfassen des zweiten Gewichts innerhalb einer Sitzung erfolgt. Eine Sitzung kann beispielsweise vorliegen, wenn an derselben Waage das erste Gewicht und das zweite Gewicht erfasst werden oder worden sind. Alternativ oder zusätzlich kann eine Sitzung beispielsweise vorliegen, wenn an derselben Waage das erste Gewicht und das zweite Gewicht innerhalb eines vorgegebenen Zeitraums erfasst werden oder worden sind. Alternativ oder zusätzlich kann vorgesehen sein, dass sich der Bediener einloggen kann, beispielsweise über ein oder das Interface, und eine Sitzung bei einem Login des Bedieners beginnen und bei einem Logout des Bedieners enden kann.

Es kann vorgesehen sein, dass das Verfahren, und/oder mindestens einer, mehrere oder alle Schritte des Verfahrens, Computer-implementiert sein kann. Einer, mehrere oder alle Schritte des Verfahrens können von einem System und/oder einer Computereinheit durchgeführt sein oder werden. Das System kann die Computereinheit aufweisen. Das System kann mindestens eine Waage zum Erfassen eines Gewichts aufweisen.

Die eindeutige ID kann in einigen Ausführungsformen durch Einlesen einer Patientenkarte erfasst werden. Die eindeutige ID kann auf der Patientenkarte gespeichert sein. In einigen Ausführungsformen kann die eindeutige ID kann auf einem Chip, einem Tag, z.B. einem RFID-Tag oder dergleichen, gespeichert sein. Die eindeutige ID kann mittels Bluetooth, RFID, NFC oder dergleichen übertragen sein oder werden. Alternativ oder zusätzlich kann die eindeutige ID in einer Datenbank, auf einem Server, einem externen Gerät oder dergleichen gespeichert sein und/oder von einer Datenbank, einem Server, einem externen Gerät oder dergleichen abgerufen sein oder werden.

Die eindeutige ID kann dem Identifizieren eines Patienten dienen. Die eindeutige ID kann ein eindeutiger Identifier (unique identifier) sein. Jeder Patient kann eine ihm zugeordnete eindeutige ID haben. Es kann vorgesehen sein, dass keine zwei Patienten dieselbe eindeutige ID haben, und/oder die eindeutige ID eines Patienten von den eindeutigen IDs jedes anderen Patienten verschieden ist. Die eindeutige ID kann ein alphanumerischer Code sein oder einen solchen aufweisen.

Das Hilfsmittel kann ein Rollstuhl, eine Gehhilfe oder eine Prothese sein oder aufweisen. Das zweite Gewicht kann ein Tara-Gewicht sein oder einem solchen entsprechen. Der Wortlaut "mit einem Hilfsmittel versehenen Patienten" kann bedeuten, dass der Patient von dem Hilfsmittel gestützt oder gehalten sein oder werden kann. Beispielsweise kann ein "mit einem Hilfsmittel versehenen Patienten" einen in einem Rollstuhl sitzenden Patienten umfassen, einen von einer Gehhilfe gestützten oder unterstützten Patienten umfassen, und/oder einen eine Prothese, z.B. eine Exoprothese, und/oder eine Orthese aufweisenden Patienten umfassen.

Nach oder bei dem Bestimmen des Patientengewichts kann ein Zuordnen des Patientengewichts zu der Gewichtsinformation und/oder der eindeutigen ID vorgesehen sein.

Mit "Zuordnen" kann ein Abspeichern, Hinterlegen und/oder Einfügen gemeint sein. Mit "Zuordnen" kann ein Verknüpfen, ein Assoziieren und/oder ein Verbinden gemeint sein. Das erfasste erste Gewicht, das erfasste zweite Gewicht, das Patientengewicht und/oder die Gewichtsinformation können Daten sein und/oder durch Daten repräsentiert werden. Das erfasste erste Gewicht, das erfasste zweite Gewicht, das Patientengewicht und/oder die Gewichtsinformation können abgespeichert sein oder werden, und/oder abspeicherbar sein, beispielsweise in einer Datenbank oder einem Datenspeicher einer Computereinheit und/oder eines Servers.

In einigen Ausführungsformen kann die Gewichtsinformation eines oder mehrere erfasste Gewichte umfassen, und/oder numerische Werte eines oder mehrerer erfasster Gewichte der Gewichtsinformation zugeordnet sein oder werden. In einigen Ausführungsformen kann die Gewichtsinformation ein Array, ein Container, oder dergleichen, für einen oder mehrere Werte sein oder aufweisen. Die Gewichtsinformation kann eine hierarchische Struktur haben und/oder hierarchisch aufgebaut sein. Die Gewichtsinformation kann eine Baumstruktur haben. In einigen Ausführungsformen kann die Gewichtsinformation Teil eines hierarchischen Datensatzes, und/oder einer hierarchischen Datenstruktur sein. Die Gewichtsinformation kann Teil einer Baumstruktur sein.

Die Gewichtsinformation kann einen Zeitstempel haben. Das erste Gewicht und/oder das zweite Gewicht, und/oder das Patientengewicht, kann einen Zeitstempel haben. Der Zeitstempel kann einen oder mehrere Zeitpunkte repräsentieren, beispielsweise eine Uhrzeit und/oder ein Datum, zu dem die jeweilige Datei, Daten, Information, Gewicht, und/oder Messung erfasst, erstellt, hinterlegt, aktualisiert, entfernt und/oder geändert worden ist.

Vor dem Erfassen des zweiten Gewichts kann nochmals die eindeutige ID des Patienten erfasst werden. Die eindeutige ID des Patienten kann durch Einlesen einer oder der Patientenkarte nochmals erfasst sein oder werden. Es kann vorgesehen sein, dass das nochmalige Erfassen der eindeutigen ID auf gleiche Art wie das Erfassen der eindeutigen ID erfolgen kann. In einigen Ausführungsformen kann aber auch das nochmalige Erfassen der eindeutigen ID auf unterschiedliche Art wie das Erfassen der eindeutigen ID erfolgen. Beispielsweise kann die eindeutige ID zunächst über eine Patientenkarte oder dergleichen erfasst werden, und die eindeutige ID nochmals über Abrufen von einer Datenbank, einem Server, einem externen Gerät oder dergleichen erfolgen.

Zwischen dem Erfassen des ersten Gewichts und dem Erfassen des zweiten Gewichts kann ein zeitlicher Abstand liegen. Der zeitliche Abstand kann beispielsweise einige Minuten oder einige Stunden, und/oder mehr als eine Minute, mehr als eine Stunde und/oder mehr als ein Tag betragen. Es kann vorgesehen sein, dass das zweite Gewicht nicht unmittelbar nach dem ersten Gewicht erfasst wird.

Wird beispielsweise eine Waage zum Erfassen des ersten Gewichts und des zweiten Gewichts verwendet, so kann zwischen dem Erfassen des ersten Gewichts und dem Erfassen des zweiten Gewichts die Waage zum Erfassen eines anderen oder weiteren Gewichts verwendet sein oder werden. Es kann vorgesehen sein, dass das erste Gewicht und das zweite Gewicht von derselben Waage erfasst sein oder werden können, und/oder dieselbe Waage zum Erfassen des ersten Gewichts und des zweiten Gewichts verwendet werden kann.

Durch das Einlesen der eindeutigen ID vor dem Erfassen des ersten Gewichts, und/oder vor dem Erfassen des zweiten Gewichts, kann sichergestellt sein oder werden, dass das erste Gewicht und/oder das zweite Gewicht dem Patienten zugeordnet werden. Beispielsweise können die jeweiligen Gewichte dem richtigen Patienten durch das Erfassen seiner eindeutigen ID auch dann richtig zugeordnet sein oder werden, wenn in der Zwischenzeit eines oder mehrere andere oder weitere Gewichte erfasst worden sind oder werden.

Das erste Gewicht kann mit einer ersten Waage und das zweite Gewicht mit einer zweiten Waage erfasst werden. Es kann vorgesehen sein, dass, die erste Waage die eindeutige ID vor dem Erfassen des ersten Gewichts erfassen kann. Es kann vorgesehen sein, dass die die zweite Waage die eindeutige ID vor dem Erfassen des zweiten Gewichts nochmals erfassen kann. Die erste Waage und/oder die zweite Waage kann beispielsweise eine Leseeinheit zum Einlesen einer Patientenkarte aufweisen.

In einigen Ausführungsformen können die erste Waage und die zweite Waage räumlich voneinander getrennt sein, beispielsweise in verschiedenen Räumen und/oder Gebäuden angeordnet sein. In einigen Ausführungsformen kann die zweite Waage für ein Wiegen ausschließlich des Hilfsmittels vorgesehen, eingerichtet, konstruiert, geeignet und/oder angeordnet sein.

Eine Waage, die erste Waage und/oder die zweite Waage kann ein Interface, beispielsweise ein Bedienfeld und/oder ein Touchdisplay oder dergleichen haben, über das ein Bediener oder Benutzer, beispielsweise ein Arzt, ein Pfleger, ein Angehöriger des Patienten, der Patient selbst, oder dergleichen, die Waage bedienen und/oder ansteuern kann. Über das Interface kann der Bediener beispielsweise das Erfassen des ersten Gewichts und/oder des zweiten Gewichts auslösen und/oder starten. In einigen Ausführungsformen kann die erste Waage und/oder die zweite Waage, und/oder das Interface, eine Taste, einen Knopf oder dergleichen haben, die ein Erfassen des ersten Gewichts auslösen kann, und/oder eine weitere Taste, Knopf oder dergleichen haben, die ein Erfassen des zweiten Gewichts auslösen kann.

Die Waage, die erste Waage, die zweite Waage, und/oder das Interface kann ein Display oder dergleichen aufweisen, über das dem Bediener beispielsweise eine Rückmeldung über das Erfassen des ersten Gewichts, das erfasst erste Gewicht, die eindeutige ID, Patienteninformationen, und/oder eine oder mehrere der eindeutigen ID zugeordnete Gewichtsinformationen, oder dergleichen, angezeigt werden können.

In einigen Ausführungsformen kann vorgesehen sein, dass das erste Gewicht nur erfasst werden kann, wenn vorher die eindeutige ID erfasst worden ist. Beispielsweise kann vorgesehen sein, dass der Bediener zunächst die eindeutige ID, z.B. mittels einer Patientenkarte, bereitstellt, z.B. an oder mit einer oder der Waage und/oder dem Lesegerät einliest, woraufhin das Erfassen des ersten Gewichts freigegeben wird. Daraufhin kann die Waage das erste Gewicht erfassen. Alternativ oder zusätzlich kann z.B. vorgesehen sein, dass der Bediener die eindeutige ID, und/oder den Patienten, für den das erste Gewicht erfasst werden soll, auswählt, beispielsweise über das Interface. Beispielsweise kann dem Bediener eine Liste von eindeutigen IDs und/oder Patienten präsentiert werden, aus der der Bediener die entsprechende eindeutige ID und/oder Patienten auswählt, für den das erste Gewicht erfasst werden soll. In einigen Ausführungsformen kann vorgesehen sein, dass das erste Gewicht automatisch nach Erfassen der eindeutigen ID erfasst wird. In einigen Ausführungsformen kann vorgesehen sein, dass mit Erfassen der eindeutigen ID ein Erfassen des ersten Gewichts ausgelöst wird, bevorzugt selbständig durch die Waage und/oder das System erfolgt und/oder ausgelöst wird. In einigen Ausführungsformen kann vorgesehen sein, dass das zweite Gewicht nur erfasst werden kann, wenn vorher die eindeutige ID erfasst worden ist. Beispielsweise kann vorgesehen sein, dass der Bediener zunächst die eindeutige ID, z.B. mittels einer Patientenkarte, bereitstellt, z.B. an oder mit einer oder der Waage und/oder dem Lesegerät einliest, woraufhin das Erfassen des ersten Gewichts freigegeben wird. Daraufhin kann die Waage das zweite Gewicht erfassen. Alternativ oder zusätzlich kann z.B. vorgesehen sein, dass der Bediener die eindeutige ID, und/oder den Patienten, für den das zweite Gewicht erfasst werden soll, auswählt, beispielsweise über das Interface. Beispielsweise kann dem Bediener eine Liste von eindeutigen IDs und/oder Patienten präsentiert werden, aus der der Bediener die entsprechende eindeutige ID und/oder Patienten auswählt, für den das zweite Gewicht erfasst werden soll.

In einigen Ausführungsformen kann vorgesehen sein, dass das zweite Gewicht automatisch nach Erfassen der eindeutigen ID erfasst wird. In einigen Ausführungsformen kann vorgesehen sein, dass mit Erfassen der eindeutigen ID ein Erfassen des zweiten Gewichts ausgelöst wird, bevorzugt selbständig durch die Waage, zweite Waage und/oder das System erfolgt und/oder ausgelöst wird. Es kann vorgesehen sein, dass dabei geprüft wird, ob bereits ein der eindeutigen ID zugeordnetes erstes Gewicht, und/oder ein einer Gewichtsinformation zugeordnetes und/oder mit der eindeutigen ID verknüpftes erstes Gewicht erfasst worden ist. In einigen Ausführungsformen kann zweite Gewicht nach erfassen der eindeutigen ID automatisch erfasst sein oder werden, wenn die Prüfung erfolgreich war, und/oder ein bereits ein der eindeutigen ID zugeordnetes erstes Gewicht, und/oder ein einer Gewichtsinformation zugeordnetes und/oder mit der eindeutigen ID verknüpftes erstes Gewicht vorliegt.

Mit "automatisch erfassen" und/oder "selbstständig erfolgt und/oder ausgelöst" kann gemeint sein, dass nach dem Erfassen der eindeutigen ID kein weiterer Input eines Benutzers oder Bedieners erforderlich ist, sein kann und/oder sein muss, um ein erstes Gewicht und/oder zweites Gewicht zu erfassen.

Alternativ oder zusätzlich kann das Prüfen, ob die Gewichtsinformation eine vollständige Gewichtsinformation ist, ein Prüfen, ob das zweite Gewicht größer als ein erster Schellwert und/oder kleiner als ein zweiter Schwellwert ist, umfassen. In einigen Ausführungsformen können der erste Schwellwert und/oder der zweite Schwellwert von dem Hilfsmittel abhängen. In einigen Ausführungsformen kann z.B. vorgesehen sein, dass der Bediener vor, bei oder nach dem Erfassen des ersten Gewichts und/oder zweiten Gewichts den Typ, die Art, das Modell oder dergleichen des Hilfsmittels auswählen oder vorgeben kann, und die entsprechenden Schwellwerte in Abhängigkeit der Auswahl gewählt sind oder werden. Beispielsweise kann der erste Schwellwert 0,2 kg bis 0,5 kg betragen und/oder der zweite Schwellwert 1 kg bis 5 kg betragen, wenn das Hilfsmittel eine Krücke oder ein Krückenpaar ist oder aufweist. Beispielsweise kann der erste Schwellwert 10 kg bis 20 kg betragen und/oder der zweite Schwellwert 20 kg bis 40 kg betragen, wenn das Hilfsmittel ein Rollstuhl ist oder aufweist. Alternativ oder zusätzlich kann ein RFID-Tag oder dergleichen, der die entsprechenden Informationen umfasst, z.B. ein RFID-Tag des Hilfsmittels, eingelesen sein oder werden. Damit kann sichergestellt und/oder von dem Verfahren und/oder System überprüft sein oder werden, dass bzw. ob das erfasste zweite Gewicht mit einem Gewicht des Hilfsmittels im Wesentlichen übereinstimmt und/oder zumindest plausibel ist.

In einigen Ausführungsformen kann vorgesehen sein, dass das erste Gewicht als Nach-Behandlungs-Messung markiert wird, wenn eine Behandlung, bevorzugt eine Dialysebehandlung, vor dem Erfassen des ersten Gewichts abgeschlossen wurde. Alternativ oder zusätzlich kann in einigen Ausführungsformen vorgesehen sein, dass das erste Gewicht als Nach-Behandlungs-Messung markiert wird, wenn eine oder die Behandlung, bevorzugt eine Dialyse-Behandlung, in einem vorgegebenen Zeitabstand, bevorzugt mehr als zwei Stunden, begonnen wurde und beim Erfassen des ersten Gewichts noch nicht abgeschlossen ist.

Die Behandlung kann dem Patienten und/oder der eindeutigen ID zugeordnet sein. Daten und/oder Informationen der Behandlung können hinterlegt sein oder werden, und zum Überprüfen, ob die Behandlung vor dem Erfassen des ersten Gewichts abgeschlossen wurde oder ist, abgerufen werden. Die Daten und/oder Informationen der Behandlung können mit der eindeutigen ID verknüpft sein.

Alternativ oder zusätzlich kann in einigen Ausführungsformen vorgesehen sein, dass das erste Gewicht als Nach-Behandlungs-Messung markiert wird, wenn ein Vor-Behandlungs-Messung vorliegt, bevorzugt ein als Vor-Behandlungs-Messung markiertes erstes Gewicht vorliegt.

Es kann vorgesehen sein, dass das erste Gewicht als Vor-Behandlungs-Messung markiert werden kann, wenn das erste Gewicht nicht als Nach-Behandlungs-Messung markiert wird und/oder worden ist.

Alternativ oder zusätzlich kann in einigen Ausführungsformen vorgesehen sein, dass nach dem Erfassen des ersten Gewichts geprüft werden kann, ob eine Information zu einer Behandlung, bevorzugt einer Dialysebehandlung, vorliegt, wobei das erste Gewicht als Nach-Behandlungs-Messung markiert werden kann, wenn das Erfassen des ersten Gewichts nach der Behandlung erfolgt ist. Es kann vorgesehen sein, dass das erste Gewicht als Vor-Behandlungs-Messung markiert werden kann, wenn das Erfassen des ersten Gewichts vor der Behandlung erfolgt ist. In einigen Ausführungsformen kann das erste Gewicht als Nach-Behandlungs-Messung markiert werden, wenn das Erfassen des ersten Gewichts mehr als ein vorgegebener Zeitabstand nach Beginn der Behandlung erfolgt ist. In einigen Ausführungsformen kann das erste Gewicht als Nach-Behandlungs-Messung markiert werden, wenn das Erfassen des ersten Gewichts mehr als ein vorgegebener Zeitabstand nach Ende der Behandlung erfolgt ist. Der vorgegebene Zeitabstand kann mehr als zwei Stunden betragen.

Die Informationen der Behandlung können ein Datum und/oder eine Uhrzeit der Behandlung, des Beginns der Behandlung und/oder des Endes der Behandlung, umfassen. Die Behandlung kann eine geplante Behandlung sein. Eine geplante Behandlung kann eine zum Zeitpunkt des Prüfens in der Zukunft liegende Behandlung sein. Die Behandlung kann eine stattgefundene Behandlung sein. Eine stattgefundene Behandlung kann eine zum Zeitpunkt des Prüfens in der Vergangenheit liegende Behandlung sein. Die Behandlung kann die zum Zeitpunkt des Prüfens zuletzt durchgeführte Behandlung sein.

Die Information zur Behandlung kann beispielsweise in einer Datenbank oder einem Datenspeicher bereitgestellt sein oder werden, und/oder mit dem Patienten, der Gewichtsinformation und/der der eindeutigen ID verknüpft und/oder dieser zugeordnet sein oder werden.

Es kann vorgesehen sein, dass der eindeutigen ID und/oder der Gewichtsinformation des Patienten ein Sollgewicht des Patienten zugeordnet sein kann, und/oder mit einem solchen verknüpft sein kann. Das Sollgewicht kann einem Sollgewicht des Patienten nach einer oder der Behandlung, z.B. nach einer geplanten Behandlung entsprechen. In einigen Ausführungsformen kann das Sollgewicht alternativ oder zusätzlich eine vorliegende Information der Behandlung bzw. Behandlungsinformation sein.

Das Sollgewicht kann ein anzustrebendes Gewicht sein, das der Patient nach der Behandlung, z.B. Dialyse, beispielsweise im Wesentlichen unmittelbar nach der Behandlung bzw. Dialyse, haben kann oder haben soll. Das Sollgewicht kann z.B. ein "Trockengewicht" eines Dialyse-Patienten sein, oder einem solchen entsprechen.

Es kann vorgesehen sein, dass ein oder das Prüfen, ob die Gewichtsinformation eine vollständige Gewichtsinformation ist, ein Prüfen, ob Sollgewicht - (erstes Gewicht - zweites Gewicht) ≤ ΔG ist, umfasst. Das Prüfen kann erfolgreich sein, wenn Sollgewicht - (erstes Gewicht - zweites Gewicht) ≤ ΔG ist. Es kann vorgesehen sein, dass ΔG ≥ 0 ist. In einigen Ausführungsformen kann 1 kg ≤ ΔG ≤ 10 kg sein. Das erste Gewicht kann dabei als Vor-Behandlungs-Messung markiert sein, und/oder das Prüfen kann durchgeführt sein oder werden, wenn das erste Gewicht als Vor-Behandlungs-Messung markiert ist. Es kann damit geprüft sein oder werden, ob der Patient an Gewicht zugenommen hat, z.B. seit einer vorherigen Behandlung und/oder Dialyse, beispielsweise insbesondere Flüssigkeitsablagerungen und/oder Wasserablagerungen vorliegen.

Alternativ oder zusätzlich kann ein oder das Prüfen, ob die Gewichtsinformation eine vollständige Gewichtsinformation ist, ein Prüfen, ob (erstes Gewicht - zweites Gewicht) - Sollgewicht ≤ ΔG ist, umfassen. Das Prüfen kann erfolgreich sein, wenn (erstes Gewicht - zweites Gewicht) - Sollgewicht ≤ ΔG ist. Es kann vorgesehen sein, dass ΔG ≥ 0 ist. In einigen Ausführungsformen kann 1 kg ≤ ΔG ≤ 10 kg sein. Das erste Gewicht kann dabei als Vor-Behandlungs-Messung markiert sein, und/oder das Prüfen kann durchgeführt sein oder werden, wenn das erste Gewicht als Vor-Behandlungs-Messung markiert ist. Es kann damit geprüft sein oder werden, ob der Patient an Gewicht zugenommen hat, z.B. seit einer vorherigen Behandlung und/oder Dialyse, beispielsweise insbesondere Flüssigkeitsablagerungen und/oder Wasserablagerungen vorliegen.

Alternativ oder zusätzlich kann ein oder das Prüfen, ob die Gewichtsinformation eine vollständige Gewichtsinformation ist, ein Prüfen, ob der Betrag |Sollgewicht - (erstes Gewicht - zweites Gewicht)| ≤ ΔG ist, umfassen. Das Prüfen kann erfolgreich sein, wenn |Sollgewicht - (erstes Gewicht - zweites Gewicht)| ≤ ΔG ist. Es kann vorgesehen sein, dass ΔG ≥ 0 sein kann. In einigen Ausführungsformen kann 0,1 kg ≤ ΔG ≤ 1 kg sein. Das erste Gewicht kann dabei als Nach-Behandlungs-Messung markiert sein, und/oder das Prüfen kann durchgeführt sein oder werden, wenn das erste Gewicht als Nach-Behandlungs-Messung markiert ist. Es kann damit z.B. geprüft sein oder werden, ob die Behandlung bzw. Dialyse ein vorgegebenes UF-Volumen entfernt hat, und/oder ob der Patient nach der Behandlung im wesentlichen sein Sollgewicht, z.B. Trockengewicht, aufweist oder erreicht hat.

Ein vor dem Erfassen des ersten Gewichts ermitteltes vorheriges Patientengewicht kann vorliegen und/oder der eindeutigen ID, und/oder einer weiteren Gewichtsinformation, zugeordnet sein. Das vorherige Patientengewicht kann ein vor dem Erfassen des ersten Gewichts und/oder nach einer vorherigen Behandlung ermitteltes vorheriges Patientengewicht sein oder aufweisen. Das vorherige Patientengewicht kann bei einer vorherigen Durchführung des Verfahrens ermittelt worden sein. Das vorherige Patientengewicht kann mit der eindeutigen ID des Patienten verknüpft sein.

Ein oder das Prüfen, ob die Gewichtsinformation eine vollständige Gewichtsinformation ist, kann ein Prüfen, ob (erstes Gewicht - zweites Gewicht) - vorheriges Patientengewicht ≥ ΔG ist, umfassen. Das Prüfen kann erfolgreich sein, wenn (erstes Gewicht - zweites Gewicht) - vorheriges Patientengewicht ≥ ΔG ist. Es kann vorgesehen sein, dass ΔG ≥ 0 ist. In einigen Ausführungsformen kann 1 kg ≤ ΔG ≤ 10 kg sein. Das erste Gewicht kann dabei als Vor-Behandlungs-Messung markiert sein, und/oder das Prüfen kann durchgeführt sein oder werden, wenn das erste Gewicht als Vor-Behandlungs-Messung markiert ist. Es kann damit geprüft sein oder werden, ob der Patient seit einer vorherigen Behandlung und/oder Dialyse, zugenommen hat, beispielsweise insbesondere Flüssigkeitsablagerungen und/oder Wasserablagerungen vorliegen.

In einigen Ausführungsformen können mehrere vor dem Erfassen des ersten Gewichts erfasste vorherige erste Gewichte und vorherige zweite Gewichte vorliegen und/oder bei einer oder mehrerer vorheriger Durchführungen des Verfahrens erfasst und/oder ermittelt worden sein. Es kann vorgesehen sein, einen Mittelwert der erfassten vorherigen ersten Gewichte und einen Mittelwert der zweiten Gewichte, z.B. vorherig erfasste zweite Gewichte, zu bestimmen, und eine Referenzdifferenz als Differenz der beiden Mittelwerte zu ermitteln. Alternativ oder zusätzlich können mehrere erfasste Differenzen von vorherig erfassten ersten Gewichten zu entsprechenden zweiten Gewichten, z.B. vorherig erfassten zweiten Gewichten vorliegen und/oder bei einer oder mehrerer vorheriger Durchführungen des Verfahrens erfasst und/oder ermittelt worden sein. Es kann vorgesehen sein, einen Mittelwert der erfassten vorherigen Differenzen als Referenzdifferenz zu bestimmen. Der oder die Mittelwerte können einen arithmetischen Mittelwert, geometrischen Mittelwert, und/oder harmonischen Mittelwert umfassen. Alternativ oder zusätzlich können zur Bestimmung der Referenzdifferenz auch andere geeignete statistische Maße wie z.B. Median oder Mode, bestimmt sein oder werden.

Ein oder das Prüfen, ob die Gewichtsinformation eine vollständige Gewichtsinformation ist, kann ein Prüfen, ob A ≤ (erstes Gewicht - zweites Gewicht) / Referenzdifferenz ≤ B ist, umfassen. Das Prüfen kann erfolgreich sein, wenn A ≤ (erstes Gewicht - zweites Gewicht) / Referenzdifferenz ≤ B ist. In einigen Ausführungsformen kann A = 1 - x und B = 1 + x mit 0 ≤ x ≤ 0,1 sein. Es kann damit z.B. geprüft sein oder werden, ob das entfernte UF-Volumen im Wesentlichen einem "historisch gemittelten" UF-Volumen, und/oder bisher üblichen UF-Volumen entspricht. Die Referenzdifferenz kann dabei eine übliche, vorgegebene und/oder durchschnittliche Differenz des ersten und zweiten Gewichts entsprechen. In einigen Ausführungsformen kann die Referenzdifferenz einem Sollgewicht, z.B. einem Trockengewicht, des Patienten entsprechen. Die Referenzdifferenz kann von dem Patienten abhängig sein. Die Referenzdifferenz kann der eindeutigen ID des Patienten zugeordnet und/oder mit dieser verknüpft sein.

Das Prüfen, ob die Gewichtsinformation eine vollständige Gewichtsinformation ist, kann aus einer Kombination zweier oder mehrerer vorstehend beschriebener Prüfschritte oder Prüfverfahren entsprechen, und/oder eine Kombination zweier oder mehrerer vorstehend beschriebener Prüfschritte oder Prüfverfahren aufweisen.

Das Prüfen, ob die Gewichtsinformation eine vollständige Gewichtsinformation ist, kann vor dem Bestimmen des Patientengewichts durchgeführt werden. Es kann vorgesehen sein, dass das Patientengewicht nur dann bestimmt wird, wenn die Gewichtsinformation als vollständige Gewichtsinformation markiert ist. Alternativ oder zusätzlich kann, wenn die Gewichtsinformation als unvollständige Gewichtsinformation markiert ist, einer, mehrere oder alle der Schritte a) - e) des Verfahrens wiederholt werden. Es kann vorgesehen sein, mindestens die Schritte b) und c) des Verfahrens zu wiederholen.

In einigen Ausführungsformen kann das zweite Gewicht (entsprechend einer oder der Messung des Gewichts des Hilfsmittels, z.B. einer Gehhilfe) sowohl auf das Vor-Dialyse-Gewicht als auch auf das Nach-Dialyse-Gewicht angewendet werden. Es kann vorgesehen sein, dass bei Wiederholungen der Messungen und/oder einer oder mehrerer Schritte des Verfahrens, insbesondere z.B. Wiederholung mindestens der Schritte b) und c) des Verfahrens, die zweite Messung nicht wiederholt werden muss.

Dies kann dem Grundgedanken, dass das Hilfsmittel während der Behandlung des Patienten nicht leichter oder schwerer wird, sondern nur und/oder höchstens im Intervall zwischen zwei Behandlungen, z.B. Dialysen, entsprechen.

Es kann vorgesehen sein, dass das zweite Gewicht außerhalb der Waage gespeichert und/oder hinterlegt sein oder werden kann, z.B. in einer externen Datenbank, externen Datenspeicher, externen Gerät. Das hinterlegte und/oder gespeicherte zweite Gewicht kann mit der eindeutigen ID verknüpft, assoziiert, verbunden, und/oder zugeordnet sein oder werden.

Es kann vorgesehen sein, dass, wenn die Gewichtsinformation als unvollständige Gewichtsinformation markiert ist, ein Hinweis und/oder eine Warnung ausgegeben werden kann. Der Hinweis und/oder die Warnung kann das erste Gewicht, das zweite Gewicht und/oder einen, mehrere oder alle der Gewichtsinformation zugeordneten Informationen, erfassten Gewichte und/oder Werte umfassen. Der Hinweis kann eine Aufforderung umfassen, nochmals ein erstes und/oder zweites Gewicht zu erfassen. Die Warnung kann anzeigen oder mitteilen, dass eines, mehrere oder alle erfassten Gewichte unplausibel oder fehlerhaft sind, und/oder dass die Gewichtsinformation unvollständig ist.

In einigen Ausführungsformen kann, wenn die Gewichtsinformation als unvollständige Gewichtsinformation markiert ist und/oder das Prüfen, ob die Gewichtsinformation eine vollständige Gewichtsinformation ist, unerfolgreich war, das zweite Gewicht als nicht plausibel oder dergleichen markiert sein oder werden. Es kann vorgesehen sein, das zweite Gewicht der Gewichtsinformation zugeordnet zu belassen, auch wenn es als nicht plausibel oder dergleichen markiert ist. Alternativ kann vorgesehen sein, als nicht plausibel markierte Gewichte zu löschen oder zu entfernen, aus der Gewichtsinformation zu löschen oder zu entfernen, und/oder die Zuordnung und/oder Verknüpfung zu entfernen oder zu lösen. In einigen Ausführungsformen kann der Bediener alternativ oder zusätzlich aufgefordert sein oder werden, nochmals ein zweites Gewicht zu erfassen. Anschließend kann nochmals mit dem bereits erfassten ersten Gewicht und dem nochmals erfassten zweiten Gewicht geprüft sein oder werden, ob die Gewichtsinformation eine vollständige Gewichtsinformation ist.

Nach dem Bestimmen des Patientengewichts kann ein Hilfsmittelgewicht als das zweite Gewicht bestimmt werden, und das Hilfsmittelgewicht der eindeutigen ID und/oder der Gewichtsinformation zugeordnet werden, und/oder in einer Datenbank gespeichert sein oder werden. Es kann vorgesehen sein, das Hilfsmittelgewicht zu einem oder dem erfassten zweiten Gewicht zu setzten. Alternativ oder zusätzlich kann ein der eindeutigen ID und/oder der Gewichtsinformation zugeordnetes, und/oder in einer Datenbank gespeichertes, Hilfsmittelgewicht durch das zweite Gewicht aktualisiert werden. Beispielsweise kann ein bereits vorliegendes Hilfsmittelgewicht durch oder mit dem zweiten Gewicht überschrieben sein oder werden.

Es kann vorgesehen sein, dass das Verfahren mindestens einmal wiederholt werden kann, so dass eine zweite Gewichtsinformation erfasst werden kann und/oder ein zeitlicher Verlauf des ersten Gewichts, des zweiten Gewichts und/oder des Patientengewichts erfasst und/oder bestimmt werden kann. Damit kann das Gewicht des Patienten bzw. das Patientengewicht überwachbar sein.

In einigen Ausführungsformen kann bei mindestens einer Wiederholung des Verfahrens anstelle des Erfassens des zweiten Gewichts durch Wiegen das zweite Gewicht dem zweiten Gewicht einer vorherigen Durchführung des Verfahrens entsprechen und/oder zu diesem gesetzt werden. Die vorherige Durchführung kann eine oder die unmittelbar vorherige Durchführung sein. In einigen Ausführungsformen kann das zweite Gewicht (entsprechend einer oder der Messung des Gewichts des Hilfsmittels, z.B. der Gehhilfe) sowohl auf das Vor-Dialyse-Gewicht als auch auf das Nach-Dialyse-Gewicht angewendet werden. Es kann vorgesehen sein, dass bei Wiederholungen der Messungen und/oder einer oder mehrerer Schritte des Verfahrens, insbesondere z.B. Wiederholung mindestens der Schritte b) und c) des Verfahrens, die zweite Messung nicht wiederholt werden muss, und/oder mindestens der Schritt d) und/oder mindestens das Erfassen des zweiten Gewichts entfallen kann.

Einer, mehrere oder alle Verfahrensschritte der offenbarten Verfahren können in einer unterschiedlichen Reihenfolge durchgeführt sein oder werden.

Ein weiterer Aspekt der Erfindung betrifft ein System zum Bestimmen eines Patientengewichts eines auf ein Hilfsmittel angewiesenen Patienten, wobei das System eine Computereinheit zum Bestimmen des Patientengewichts und eine Waage zum Erfassen eines ersten Gewichts, das dem Gewicht des Patienten und dem Gewicht des Hilfsmittels entspricht, und/oder eines zweiten Gewichts, das dem Gewicht des Hilfsmittels entspricht, aufweist, dadurch gekennzeichnet, dass das System dazu eingerichtet ist, ein vorstehend beschriebene computerimplementierte Verfahren durchzuführen.

Das System kann dazu verwendet werden, eines, mehrere oder alle der vorstehend beschriebenen computerimplementieren Verfahren durchzuführen. Eines, mehrere oder alle der vorstehend beschriebenen Verfahren, und/oder einer, mehrere oder alle Schritte des oder der Verfahren, können von einem entsprechenden System ausgeführt sein oder werden. Das System, die Computereinheit, und/oder die Waage kann eines, mehrere oder alle der vorstehend beschriebenen, insbesondere mit Bezug zu dem Verfahren beschriebenen, Merkmale und/oder Vorteile aufweisen.

Das System kann dazu eingerichtet sein, das erste Gewicht als Nach-Behandlungs-Messung zu markieren, wenn: eine Behandlung, bevorzugt eine Dialysebehandlung, vor dem Erfassen des ersten Gewichts abgeschlossen wurde; und/oder wenn eine Behandlung, bevorzugt eine Dialyse-Behandlung, in einem vorgegebenen Zeitabstand, bevorzugt mehr als zwei Stunden, begonnen wurde und beim Erfassen des ersten Gewichts noch nicht abgeschlossen ist; und/oder wenn ein Vor-Behandlungs-Messung vorliegt, bevorzugt ein als Vor-Behandlungs-Messung markiertes erstes Gewicht vorliegt. Das erste Gewicht kann als Vor-Behandlungs-Messung markiert werden, wenn das erste Gewicht nicht als Nach-Behandlungs-Messung markiert wird.

Das System kann dazu eingerichtet sein, der eindeutigen ID und/oder der Gewichtsinformation des Patienten ein Sollgewicht des Patienten, bevorzugt ein Sollgewicht nach einer oder der Behandlung, zuzuordnen, und wobei das Prüfen, ob die Gewichtsinformation eine vollständige Gewichtsinformation ist, umfassen kann: Prüfen, wenn das erste Gewicht als Vor-Behandlungs-Messung markiert ist, ob Sollgewicht - (erstes Gewicht - zweites Gewicht) ≤ ΔG ist, wobei bevorzugt ΔG ≥ 0 ist, besonders bevorzugt 1 kg ≤ ΔG ≤ 10 kg ist; und/oder Prüfen, wenn das erste Gewicht als Vor-Behandlungs-Messung markiert ist, ob (erstes Gewicht - zweites Gewicht) - Sollgewicht ≤ ΔG ist, wobei bevorzugt ΔG ≥ 0 ist, besonders bevorzugt 1 kg ≤ ΔG ≤ 10 kg ist; und/oder Prüfen, wenn das erste Gewicht als Nach-Behandlungs-Messung markiert ist, ob der Betrag |Sollgewicht - (erstes Gewicht - zweites Gewicht)| ≤ ΔG ist, wobei bevorzugt ΔG ≥ 0 ist, besonders bevorzugt 0.1 kg ≤ ΔG ≤ 1 kg ist. Wenn das Prüfen erfolgreich war, kann die Gewichtsinformation als vollständige Gewichtsinformation markiert werden, und wenn das Prüfen unerfolgreich war, kann die Gewichtsinformation als unvollständige Gewichtsinformation markiert werden.

Bei dem System kann ein vor dem Erfassen des ersten Gewichts ermitteltes vorheriges Patientengewicht des Patienten, bevorzugt ein vor dem Erfassen des ersten Gewichts und nach einer vorherigen Behandlung ermitteltes vorheriges Patientengewicht des Patienten, vorliegen und/oder der eindeutigen ID zugeordnet sein, und das Prüfen, ob die Gewichtsinformation eine vollständige Gewichtsinformation ist, ein Prüfen, wenn das erste Gewicht als Vor-Behandlungs-Messung markiert ist, ob (erstes Gewicht - zweites Gewicht) - vorheriges Patientengewicht ≥ ΔG ist, umfasse, wobei bevorzugt ΔG ≥ 0 sein kann, besonders bevorzugt 1 kg ≤ ΔG ≤ 10 kg sein kann.

Alternativ oder zusätzlich können bei dem System mehrere vor dem Erfassen des ersten Gewichts erfasste vorherige erste Gewichte und vorherige zweite Gewichte, und/oder vorherig erfasste Differenzen (vorherig erfasstes erstes Gewicht - vorherig erfasstes zweites Gewicht) vorliegen, und eine Referenzdifferenz als Differenz der mittleren Abweichung der vorherig erfassten ersten Gewichte und der mittleren Abweichung der vorherig erfassten zweiten Gewichte, und/oder als eine mittlere Abweichung der vorherig erfassten Differenzen, bestimmt sein oder werden, und das Prüfen ein Prüfen, ob A ≤ (erstes Gewicht - zweites Gewicht) / Referenzdifferenz ≤ B ist, umfassen, wobei bevorzugt A = 1 - x und B = 1 + x mit 0 ≤ x ≤ 0,1 sein kann.

Die Waage kann dazu eingerichtet sein, eine eindeutige ID des Patienten zu erfassen. Beispielsweise kann die Waage eine Leseeinheit aufweisen, die zum Einlesen einer Patientenkarte eingerichtet sein kann. Alternativ oder zusätzlich kann die eindeutige ID aber auch z.B. durch NFC, Bluetooth oder dergleichen an die Waage und/oder die Computereinheit übermittelt sein oder werden.

Die Waage kann eine Gewichtsbestimmungseinheit aufweisen, die einen Wert eines Gewichts erfassen kann. Die Waage und/oder die Gewichtsbestimmungseinheit kann mit der Computereinheit zum Datenaustausch verbunden sein. Die Waage und/oder die Gewichtsbestimmungseinheit kann erfasste Gewichte an die Computereinheit übermitteln.

In einigen Ausführungsformen kann die Waage die Computereinheit aufweisen. In einigen Ausführungsformen kann eine oder die Computereinheit alternativ oder zusätzlich ein externes Gerät sein, z.B. ein externer PC, Laptop und/oder Server. In einigen Ausführungsformen kann alternativ oder zusätzlich ein oder das externe Gerät, z.B. ein externer PC, Laptop und/oder Server, die Computereinheit sein oder aufweisen.

Die Computereinheit kann einen Speicher bzw. einen Datenspeicher und/oder eine Datenbank aufweisen, in dem oder in der Daten hinterlegt oder abgerufen werden können. In dem Datenspeicher und/oder der Datenbank können z.B. erste Gewichte, zweite Gewichte, Gewichtsinformationen, Patientengewichte, eindeutige IDs, Behandlungsinformationen, Sollgewichte, und dergleichen, und/oder mit diesen verknüpfte oder diesen zugeordnete weitere Daten hinterlegt sein oder werden.

In einigen Ausführungsformen kann die Computereinheit mit einem Server und/oder einem externen Gerät verbunden sein. Die Computereinheit kann dazu eingerichtet sein, Daten von dem Server und/oder dem externen Gerät zu empfangen und/oder an Server und/oder externes Gerät zu kommunizieren. Beispielsweise kann die Computereinheit in einigen Ausführungsformen erste Gewichte, zweite Gewichte, Gewichtsinformationen, Patientengewichte, eindeutige IDs, Behandlungsinformationen, Sollgewichte, und dergleichen, und/oder mit diesen verknüpfte oder diesen zugeordnete weitere Daten von dem Server und/oder dem externem Gerät abrufen oder an den Server und/oder an das externe Gerät übermitteln.

Das System kann eine zweite Waage aufweisen, die dazu eingerichtet sein kann, das zweite Gewicht zu erfassen. In einigen Ausführungsformen ist die zweite Waage ausschließlich dafür vorgesehen, verwendet und/oder eingerichtet, das zweite Gewicht zu erfassen und/oder ausschließlich das Hilfsmittel zu wiegen. Die zweite Waage kann beispielsweise geeignet dimensioniert, konfiguriert, angepasst oder ausgeführt sein.

Die zweite Waage kann mit der Computereinheit zum Datenaustausch verbunden sein. In einigen Ausführungsformen kann die zweite Waage dazu eingerichtet sein, die eindeutige ID zu erfassen. Beispielsweise kann die zweite Waage eine Leseeinheit aufweisen, die zum Einlesen einer Patientenkarte eingerichtet sein kann. Alternativ oder zusätzlich kann die eindeutige ID aber auch z.B. durch NFC, Bluetooth oder dergleichen an die zweite Waage und/oder die Computereinheit übermittelt sein oder werden. In einigen Ausführungsformen kann die zweite Waage eines, mehrere oder alle Merkmale und/oder Vorteile der Waage und/oder ersten Waage aufweisen.

Die Waage, erste Waage und/oder zweite Waage kann ein Interface aufweisen, z.B. ein vorstehend beschriebenes Interface.

Die Computereinheit kann eine Datenbank aufweisen und/oder mit einer Datenbank verbunden sein. Die Computereinheit kann dazu eingerichtet sein, mit der eindeutigen ID des Patienten verknüpfte und zu verschiedenen Zeitpunkten erfasste Gewichtsinformationen und/oder zu verschiedenen Zeitpunkten bestimmte Patientengewichte in der Datenbank zu speichern, so dass ein zeitlicher Verlauf von der Computereinheit bestimmbar ist. Die Gewichtsinformationen können vollständige Gewichtsinformationen sein oder aufweisen, und/oder erfasste erste Gewichte und/oder erfasste zweite Gewichte umfassen.

Eine geplante Behandlungsdauer einer geplanten Behandlung, bevorzugt eine geplante Dialysedauer, und ein Sollgewicht des Patienten, bevorzugt ein Sollgewicht des Patienten nach der geplanten Behandlung, kann der Computereinheit bekannt und/oder von der Computereinheit abrufbar sein. Die Computereinheit kann dazu eingerichtet sein, ein geplantes UF-Volumen aus der Differenz (erstes Gewicht - zweites Gewicht) - Sollgewicht und weiter eine mittlere UF-Rate aus dem Verhältnis des geplanten UF-Volumens zu der geplanten Behandlungsdauer, d.h. geplantes UF-Volumen / geplante Behandlungsdauer, zu bestimmen. Das erste Gewicht kann eine Vor-Behandlungs-Messung sein, und/oder das Bestimmen des geplanten UF-Volumens und der mittleren UF-Rate kann durchgeführt sein oder werden, wenn das erste Gewicht eine Vor-Behandlungs-Messung ist. Das UF-Volumen kann ein Ultrafiltrations-Volumen sein oder aufweisen. Die UF-Rate kann eine Ultrafiltrations-Rate sein oder aufweisen.

Das geplante UF-Volumen kann einer bei der Behandlung, bevorzugt einer Dialysebehandlung, zu entfernenden Menge an Flüssigkeit und/oder Wasser entsprechen. Ein kg Wasser hat unter üblichen Bedingungen im Wesentlichen ein Volumen von einem Liter, so dass das Gewicht der zu entfernenden und/oder entfernten Flüssigkeit und/oder des Wassers in ein Volumen bzw. das UF-Volumen umrechenbar ist und/oder diesem entspricht. Das UF-Volumen kann die Einheit [m³], oder eine davon abgeleitete oder umgerechnete Größe und/oder Einheit, z.B. [L], haben.

Die mittlere UF-Rate (Ultrafiltration-Rate) kann einer oder der durchschnittlichen UF-Rate bei der Behandlung, z.B. einer Dialysebehandlung, entsprechen. Die mittlere UF-Rate (Ultrafiltration-Rate) kann eine vorgegebene UF-Rate oder Soll-UF-Rate bei der Behandlung, z.B. einer Dialysebehandlung, entsprechen. Die UF-Rate kann die Einheit [m³/s], oder eine Umrechnung, z.B. [L/h], haben.

Die Computereinheit kann dazu eingerichtet sein, die Bestimmung einer oder der mittleren UF-Rate bei oder nach dem Erfassen des zweiten Gewichts, und/oder dem Bestimmen des Patientengewichts, durchzuführen. Das System und/oder die Computereinheit kann dazu eingerichtet sein, einen Hinweis oder eine Warnung auszugeben, wenn eine für den Patienten charakteristische maximale UF-Rate nicht eingehalten werden kann, und/oder überschritten ist oder wird. Die charakteristische maximale UF-Rate kann eine Einheit [L/h] haben.

Die charakteristische maximale UF-Rate kann von dem Patientengewicht bzw. dem Körpergewicht des Patienten abhängen. Die charakteristische maximale UF-Rate kann eine spezifische charakteristische maximale UF-Rate sein, die aus dem Verhältnis charakteristische maximale UF-Rate / Patientengewicht bestimmt sein oder werden kann. Die mittlere UF-Rate kann eine spezifische mittlere UF-Rate sein, die als mittlere UF-Rate / Patientengewicht definiert sein kann. Die Computereinheit kann die spezifische mittlere UF-Rate bestimmen.

Die spezifische charakteristische maximale UF-Rate kann von dem Patienten unabhängig sein, und/oder patientenunabhängig angenommen sein oder werden. Die charakteristische maximale UF-Rate kann von dem Patienten, insbesondere dem Patientengewicht, abhängig sein, und/oder patientenabhängig angenommen sein oder werden.

Die charakteristische maximale UF-Rate und/oder die spezifische charakteristische maximale UF-Rate kann ein Grenzwert sein, bei deren Überschreitung ein Risiko für den Patienten bestehen, entstehen und/oder vergrößert sein kann.

Bei zu hoher UF-Rate können Nebenwirkungen oder Schädigungen des Patienten auftreten, und/oder kardiovaskuläre Risiken erhöht oder Krankenhausaufenthalte aufgrund von Herz-Kreislauf-Erkrankungen wahrscheinlicher werden. Die Grenzwerte zu hoher UF-Rate sind dabei üblicherweise von dem Patientengewicht abhängig. Die Risiken können mit steigender spezifischer UF-Rate (und/oder für einen gegebenen Patienten entsprechender mit zunehmender UF-Rate), z.B. steigender spezifischer mittlerer UF-Rate und/oder steigender mittlerer UF-Rate, bei Überschreiten des Grenzwerts zunehmen. In einigen Fällen kann z.B. der Grenzwert der spezifischen mittleren UF-Rate 10 ml/h/kg betragen. In einigen Fällen kann z.B. der Grenzwert der spezifischen mittleren UF-Rate weniger als 10 ml/h/kg betragen. In einigen Fällen kann z.B. der Grenzwert der spezifischen mittleren UF-Rate 8 ml/h/kg, oder weniger, betragen.

In einigen Ausführungsformen kann die geplante Behandlungsdauer vorgegeben sein. Damit kann die charakteristische maximale UF-Rate und/oder die spezifische charakteristische maximale UF-Rate bestimmt sein oder werden. Beispielsweise kann die Computereinheit die geplante Behandlungsdauer, die charakteristische maximale UF-Rate und/oder die spezifische charakteristische maximale UF-Rate bestimmen, und/oder abrufen. In einigen Ausführungsformen kann die geplante Behandlungsdauer, die charakteristische maximale UF-Rate und/oder die spezifische charakteristische maximale UF-Rate in einem Speicher, einer Datenbank und/oder einem Server hinterlegt sein, auf den die Computereinheit zugreifen kann. Die geplane Behandlungsdauer, die charakteristische maximale UF-Rate und/oder die spezifische charakteristische maximale UF-Rate kann der eindeutigen ID zugeordnet und/oder mit dieser verknüpft sein.

Es kann geprüft werden, ob die für den Patienten charakteristische maximale UF-Rate nicht eingehalten werden kann. In einigen Ausführungsformen kann dazu die spezifische charakteristische maximale UF-Rate bestimmt, ermittelt und/oder abgerufen sein, und mit dem Patientengewicht multipliziert sein oder werden, um damit die charakteristische maximale UF-Rate zu bestimmen. Anschließend kann die charakteristische maximale UF-Rate mit der mittleren UF-Rate verglichen sein oder werden. Ist die mittlere UF-Rate höher als die charakteristische maximale UF-Rate, so kann die charakteristische maximale UF-Rate nicht eingehalten sein.

Alternativ oder zusätzlich kann zum Prüfen, ob für den Patienten charakteristische maximale UF-Rate nicht eingehalten werden kann, die spezifische charakteristische maximale UF-Rate bestimmt, ermittelt und/oder abgerufen sein. Die spezifische mittlere UF-Rate kann durch Teilen der mittleren UF-Rate durch das Patientengewicht bestimmt sein oder werden. Anschließend kann die spezifische charakteristische maximale UF-Rate mit der spezifischen mittleren UF-Rate verglichen sein oder werden. Ist die spezifische mittlere UF-Rate höher als die spezifische charakteristische maximale UF-Rate, so kann die charakteristische maximale UF-Rate nicht eingehalten sein.

Durch die festgelegte Behandlungsdauer und das geplante UF-Volumen kann sich eine mittlere UF-Rate ergeben, die eingehalten werden muss, um innerhalb der Behandlungsdauer das geplante UF-Volumen zu entfernen, bzw. um nach der Behandlung das geplante UF-Volumen entfernt zu haben. Bei Unterschreiten der mittleren UF-Rate kann damit das geplante UF-Volumen nicht erreicht sein oder werden, bzw. kann weniger als das geplante UF-Volumen entfernt sein oder werden.

Es können damit in Abhängigkeit des Patientengewichts Fälle auftreten, bei denen die mittlere UF-Rate nicht eingehalten werden kann oder sollte, da andernfalls Risiken für den Patienten bestehen würden. Es kann in solchen Fällen bei vorgegebener Behandlungsdauer nicht das geplante UF-Volumen entfernt sein oder werden, oder sollte zumindest das geplante UF-Volumen nicht entfernt sein oder werden.

In einigen Ausführungsformen kann die mittlere UF-Rate zu der charakteristischen maximalen UF-Rate gesetzt werden, und/oder auf einen Wert kleiner der der charakteristischen maximalen UF-Rate gesetzt werden. Die derart neu bestimmte mittlere UF-Rate, und/oder verringerte mittlere UF-Rate, kann in einem oder dem Datenspeicher und/oder Datenbank hinterlegt und/oder der eindeutigen ID zugeordnet und/oder mit dieser verknüpft sein oder werden. Es kann vorgesehen sein, dass die Behandlung, z.B. Dialyse, mit der verringerten mittleren UF-Rate durchgeführt sein oder werden kann. Der Hinweis und/oder die Warnung kann entsprechend darauf hinweisen, und/oder die (ursprünglich geplante) mittlere UF-Rate, verringerte mittlere UF-Rate, charakteristische maximale UR-Rate, bzw. die entsprechenden spezifischen Raten mitteilen, z.B. aufführen. Der Hinweis und/oder die Warnung kann die geplante Behandlungsdauer und/oder das Patientengewicht mitteilen.

Durch die verringerte mittlere UF-Rate kann sich die Notwendigkeit einer längeren Behandlungsdauer (um damit das geplante UF-Volumen bei verringerter UF-Rate zu entfernen) und/oder mindestens einer weiteren Behandlung (um das noch nicht entfernte verbleibenden UF-Volumen zu entfernen) ergeben. Der Hinweis und/oder die Warnung kann darauf hinweisen, und/oder dies mitteilen.

Das System kann dazu eingerichtet sein, eine geplante Behandlung, bevorzugt eine geplante Dialyse-Behandlung, nur dann freizugeben, wenn das Patientengewicht bestimmt worden ist und/oder wenn die Gewichtsinformation eine vollständige Gewichtsinformation ist.

Alternativ oder zusätzlich kann das System dazu eingerichtet sein, eine geplante Behandlung, bevorzugt eine geplante Dialyse-Behandlung, nur dann freizugeben, wenn die charakteristische maximale UF-Rate eingehalten werden kann.

Das System kann einen Drucker aufweisen, der dazu eingerichtet sein kann, einen Bon mit einem Hinweis und/oder einer Warnung, einer oder mehrerer Gewichtsinformationen, dem erfassten ersten Gewicht, erfassten zweiten Gewicht und/oder dem Patientengewicht zu bedrucken und auszugeben.

In einigen Ausführungsformen kann eine oder die Waage, oder jede Waage, einen oder den Drucker aufweisen. Es kann vorgesehen sein, den Hinweis und/oder die Warnung unmittelbar, oder kurz nach, einer Bedienung der Waage und/oder des Interfaces auszugeben und/oder auszudrucken. In einigen Ausführungsformen kann der Drucker nach der Erfassen des ersten Gewichts, des zweiten Gewichts, dem Prüfen, ob eine vollständige Gewichsinformation vorliegt, dem Bestimmen des Patientengewichts und/oder dem Bestimmen der mittleren UF-Rate einen oder den Bon ausgeben und/oder ausdrucken, und/oder entsprechend eingerichtet sein.

Damit kann der Bediener und/oder Benutzer unmittelbar eine Rückmeldung, z.B. bezüglich der Gewichtserfassung, erhalten.

## Patentansprüche

1. Computerimplementiertes Verfahren zum Bestimmen eines Patientengewichts eines auf ein Hilfsmittel angewiesenen Patienten, mit den folgenden Schritten:
a) Erfassen einer eindeutigen ID des Patienten, bevorzugt durch Einlesen einer Patientenkarte;
b) Erfassen eines ersten Gewichts durch Wiegen des mit einem Hilfsmittel, bevorzugt einem Rollstuhl, einer Gehhilfe oder einer Prothese, versehenen Patienten, so dass das erste Gewicht der Summe aus dem Gewicht des Patienten und dem Gewicht des Hilfsmittels entspricht;
c) Erstellen einer mit der eindeutigen ID des Patienten verknüpften Gewichtsinformation, und Zuordnen des ersten Gewichts zu der Gewichtsinformation;
d) Erfassen eines zweiten Gewichts durch Wiegen des Hilfsmittels;
e) Zuordnen des zweiten Gewichts zu der Gewichtsinformation; und
f) Bestimmen des Patientengewichts des Patienten durch Subtraktion des zweiten Gewichts von dem ersten Gewicht, und bevorzugt Zuordnen des Patientengewichts zu der Gewichtsinformation und/oder der eindeutigen ID,
**dadurch gekennzeichnet, dass** das Verfahren ein Prüfen, ob die Gewichtsinformation eine vollständige Gewichtsinformation ist, umfasst, wobei das Prüfen umfasst:
- Prüfen, ob das zweite Gewicht erfasst worden ist; und/oder
- Prüfen, ob das erste Gewicht in einem zeitlichen Zusammenhang mit dem zweiten Gewicht steht, wobei bevorzugt ein zeitlicher Zusammenhang vorliegt, wenn das Erfassen des ersten Gewichts und das Erfassen des zweiten Gewichts innerhalb eines vorgegebenen Zeitintervalls, besonders bevorzugt innerhalb 24 Stunden, erfolgt; und/oder
- Prüfen, ob das erste Gewicht in einem logischen Zusammenhang mit dem zweiten Gewicht steht, wobei bevorzugt ein logischer Zusammenhang vorliegt, wenn das Erfassen des ersten Gewichts und das Erfassen des zweiten Gewichts innerhalb einer Sitzung erfolgt; und/oder
- Prüfen, ob das zweite Gewicht geringer als das erste Gewicht ist, und/oder Prüfen, ob das zweite Gewicht größer als 0,5 kg und/oder kleiner als 200 kg ist,
wobei, wenn das Prüfen erfolgreich war, die Gewichtsinformation als vollständige Gewichtsinformation markiert wird, und wenn das Prüfen unerfolgreich war, die Gewichtsinformation als unvollständige Gewichtsinformation markiert wird, wobei das Prüfen, ob die Gewichtsinformation eine vollständige Gewichtsinformation ist, vor dem Bestimmen des Patientengewichts durchgeführt wird und das Patientengewicht nur dann bestimmt wird, wenn die Gewichtsinformation als vollständige Gewichtsinformation markiert ist, und/oder wobei, wenn die Gewichtsinformation als unvollständige Gewichtsinformation markiert ist, einer, mehrere oder alle der Schritte a) - e) des Verfahrens wiederholt werden, bevorzugt mindestens die Schritte b) und c) wiederholt werden.

2. Computerimplementiertes Verfahren nach Anspruch 1, bei dem vor dem Erfassen des zweiten Gewichts nochmals die eindeutige ID des Patienten erfasst wird, bevorzugt durch Einlesen einer oder der Patientenkarte.

3. Computerimplementiertes Verfahren nach Anspruch 1 oder 2, bei dem das erste Gewicht mit einer ersten Waage und das zweite Gewicht mit einer zweiten Waage erfasst wird, wobei die erste Waage die eindeutige ID vor dem Erfassen des ersten Gewichts erfasst, und wobei bevorzugt die zweite Waage die eindeutige ID vor dem Erfassen des zweiten Gewichts nochmals erfasst.

4. Computerimplementiertes Verfahren nach einem der vorangegangenen Ansprüche 1 bis 3, wobei, wenn die Gewichtsinformation als unvollständige Gewichtsinformation markiert ist, ein Hinweis und/oder eine Warnung ausgegeben wird, wobei der Hinweis und/oder die Warnung das erste Gewicht, das zweite Gewicht und/oder einen, mehrere oder alle der Gewichtsinformation zugeordneten Informationen, erfassten Gewichte und/oder Werte umfasst.

5. Computerimplementiertes Verfahren nach einem der vorangegangenen Ansprüche, wobei nach dem Bestimmen des Patientengewichts ein Hilfsmittelgewicht als das zweite Gewicht bestimmt und das Hilfsmittelgewicht der eindeutigen ID und/oder der Gewichtsinformation zugeordnet wird, und/oder ein der eindeutigen ID und/oder der Gewichtsinformation zugeordnetes Hilfsmittelgewicht durch das zweite Gewicht aktualisiert wird.

6. Computerimplementiertes Verfahren nach einem der vorangegangenen Ansprüche, wobei das Verfahren mindestens einmal wiederholt wird, so dass eine zweite Gewichtsinformation erfasst wird und/oder ein zeitlicher Verlauf des ersten Gewichts, des zweiten Gewichts und/oder des Patientengewichts erfasst und/oder bestimmt wird, wobei bevorzugt bei mindestens einer Wiederholung des Verfahrens anstelle des Erfassens des zweiten Gewichts durch Wiegen das zweite Gewicht dem zweiten Gewicht einer vorherigen Durchführung des Verfahrens, bevorzugt der unmittelbar vorherigen Durchführung, entspricht und/oder zu diesem gesetzt wird.

7. System zum Bestimmen eines Patientengewichts eines auf ein Hilfsmittel angewiesenen Patienten, wobei das System eine Computereinheit zum Bestimmen des Patientengewichts und eine Waage zum Erfassen eines ersten Gewichts, das dem Gewicht des Patienten und dem Gewicht des Hilfsmittels entspricht, und/oder eines zweiten Gewichts, das dem Gewicht des Hilfsmittels entspricht, aufweist, wobei die Waage bevorzugt dazu weiter eingerichtet ist, eine eindeutige **ID** des Patienten zu erfassen, **dadurch gekennzeichnet, dass** das System dazu eingerichtet ist, das computerimplementierte Verfahren nach einem der vorangegangenen Ansprüche 1 bis 6 durchzuführen.

8. System nach Anspruch 7, bei dem das System weiter dazu eingerichtet ist, das erste Gewicht als Nach-Behandlungs-Messung zu markieren, wenn
- eine Behandlung, bevorzugt eine Dialysebehandlung, vor dem Erfassen des ersten Gewichts abgeschlossen wurde; und/oder
- wenn eine Behandlung, bevorzugt eine Dialyse-Behandlung, in einem vorgegebenen Zeitabstand, bevorzugt mehr als zwei Stunden, begonnen wurde und beim Erfassen des ersten Gewichts noch nicht abgeschlossen ist; und/oder
- wenn ein Vor-Behandlungs-Messung vorliegt, bevorzugt ein als Vor-Behandlungs-Messung markiertes erstes Gewicht vorliegt,
wobei das erste Gewicht als Vor-Behandlungs-Messung markiert wird, wenn das erste Gewicht nicht als Nach-Behandlungs-Messung markiert wird.

9. System nach Anspruch 7 oder 8, wobei das System dazu eingerichtet ist, der eindeutigen ID und/oder der Gewichtsinformation des Patienten ein Sollgewicht des Patienten, bevorzugt ein Sollgewicht nach einer oder der Behandlung, zuzuordnen, und wobei das Prüfen, ob die Gewichtsinformation eine vollständige Gewichtsinformation ist, umfasst:
- Prüfen, wenn das erste Gewicht als Vor-Behandlungs-Messung markiert ist, ob Sollgewicht - (erstes Gewicht - zweites Gewicht) ≤ ΔG ist, wobei bevorzugt ΔG ≥ 0 ist, besonders bevorzugt 1 kg ≤ ΔG ≤ 10 kg ist; und/oder
- Prüfen, wenn das erste Gewicht als Vor-Behandlungs-Messung markiert ist, ob (erstes Gewicht - zweites Gewicht) - Sollgewicht ≤ ΔG ist, wobei bevorzugt ΔG ≥ 0 ist, besonders bevorzugt 1 kg ≤ ΔG ≤ 10 kg ist; und/oder
- Prüfen, wenn das erste Gewicht als Nach-Behandlungs-Messung markiert ist, ob der Betrag |Sollgewicht - (erstes Gewicht - zweites Gewicht)| ≤ ΔG ist, wobei bevorzugt ΔG ≥ 0 ist, besonders bevorzugt 0.1 kg ≤ ΔG ≤ 1 kg ist,
wobei, wenn das Prüfen erfolgreich war, die Gewichtsinformation als vollständige Gewichtsinformation markiert wird, und wenn das Prüfen unerfolgreich war, die Gewichtsinformation als unvollständige Gewichtsinformation markiert wird.

10. System nach einem der vorangegangenen Ansprüche 8 oder 9, bei dem
- ein vor dem Erfassen des ersten Gewichts ermitteltes vorheriges Patientengewicht des Patienten, bevorzugt ein vor dem Erfassen des ersten Gewichts und nach einer vorherigen Behandlung ermitteltes vorheriges Patientengewicht des Patienten, vorliegt und/oder der eindeutigen ID zugeordnet ist, und wobei das Prüfen, ob die Gewichtsinformation eine vollständige Gewichtsinformation ist, ein Prüfen, wenn das erste Gewicht als Vor-Behandlungs-Messung markiert ist, ob (erstes Gewicht - zweites Gewicht) - vorheriges Patientengewicht ≥ ΔG ist, umfasst, wobei bevorzugt ΔG ≥ 0 ist, besonders bevorzugt 1 kg ≤ ΔG ≤ 10 kg ist; und/oder bei dem
- mehrere vor dem Erfassen des ersten Gewichts erfasste vorherige erste Gewichte und vorherige zweite Gewichte, und/oder vorherig erfasste Differenzen (vorherig erfasstes erstes Gewicht - vorherig erfasstes zweites Gewicht) vorliegen, und eine Referenzdifferenz als Differenz der mittleren Abweichung der vorherig erfassten ersten Gewichte und der mittleren Abweichung der vorherig erfassten zweiten Gewichte, und/oder als eine mittlere Abweichung der vorherig erfassten Differenzen, bestimmt ist oder wird, und das Prüfen ein Prüfen, ob A ≤ (erstes Gewicht - zweites Gewicht) / Referenzdifferenz ≤ B ist, umfasst, wobei bevorzugt A = 1 - x und B = 1 + x mit 0 ≤ x ≤ 0,1.

11. System nach einem der vorangegangenen Ansprüche 7 bis 10, das eine zweite Waage aufweist, die dazu eingerichtet ist, das zweite Gewicht, und bevorzugt die eindeutige ID, zu erfassen.

12. System nach einem der vorangegangenen Ansprüche 7 bis 11, bei dem die Computereinheit eine Datenbank aufweist und/oder mit einer Datenbank verbunden ist, und die Computereinheit dazu eingerichtet ist, mit der eindeutigen ID des Patienten verknüpfte und zu verschiedenen Zeitpunkten erfasste Gewichtsinformationen, bevorzugt vollständige Gewichtsinformationen, erfasste erste Gewichte, erfasste zweite Gewichte, und/oder zu verschiedenen Zeitpunkten bestimmte Patientengewichte in der Datenbank zu speichern, so dass ein zeitlicher Verlauf von der Computereinheit bestimmbar ist.

13. System nach einem der vorangegangenen Ansprüche 7 bis 12, bei dem eine geplante Behandlungsdauer einer geplanten Behandlung, bevorzugt eine geplante Dialysedauer, und ein Sollgewicht des Patienten, bevorzugt ein Sollgewicht des Patienten nach der geplanten Behandlung, der Computereinheit bekannt und/oder von der Computereinheit abrufbar ist, und wobei die Computereinheit dazu eingerichtet ist, wenn das erste Gewicht eine Vor-Behandlungs-Messung ist, ein geplantes UF-Volumen aus der Differenz (erstes Gewicht - zweites Gewicht) - Sollgewicht und weiter eine mittlere UF-Rate aus dem Verhältnis des geplanten UF-Volumens zu der geplanten Behandlungsdauer zu bestimmen.

14. System nach Anspruch 13, bei dem die Computereinheit dazu eingerichtet ist, die Bestimmung der mittleren UF-Rate bei oder nach dem Erfassen des zweiten Gewichts durchzuführen, und wobei das System dazu eingerichtet ist, einen Hinweis oder eine Warnung auszugeben, wenn eine für den Patienten charakteristische maximale UF-Rate nicht eingehalten werden kann, bevorzugt aufgrund des Patientengewichts nicht eingehalten werden kann.

15. System nach einem der vorangegangenen Ansprüche 7 bis 14, das dazu eingerichtet ist, eine geplante Behandlung, bevorzugt eine geplante Dialyse-Behandlung, nur dann freizugeben, wenn das Patientengewicht bestimmt worden ist und/oder wenn die Gewichtsinformation eine vollständige Gewichtsinformation ist.

16. System nach einem der vorangegangenen Ansprüche 7 bis 15, das einen Drucker aufweist, der dazu eingerichtet ist, einen Bon mit einem Hinweis und/oder einer Warnung, einer oder mehrerer Gewichtsinformationen, dem erfassten ersten Gewicht, erfassten zweiten Gewicht und/oder dem Patientengewicht zu bedrucken und auszugeben.

## Claims

1. Computer-implemented method for determining a patient weight of a patient in need of an adjuvant, comprising the following steps:
a) acquiring a unique ID of the patient, preferably by reading in a patient card;
b) acquiring a first weight by weighing the patient provided with an adjuvant, preferably a wheelchair, a walking aid or a prosthesis, such that the first weight corresponds to the sum of the weight of the patient and the weight of the adjuvant;
c) creating weight information linked to the unique ID of the patient, and assigning the first weight to the weight information;
d) acquiring a second weight by weighing the adjuvant;
e) assigning the second weight to the weight information; and
f) determining the patient weight of the patient by subtracting the second weight from the first weight, and preferably assigning the patient weight to the weight information and/or the unique ID,
**characterized in that** the method comprises checking whether the weight information is a complete weight information, wherein the checking comprises:
- checking whether the second weight has been detected; and/or
- checking whether the first weight is in a temporal relationship with the second weight, wherein there is preferably a temporal relationship if the first weight is acquired and the second weight is acquired within a predefined time interval, particularly preferably within 24 hours; and/or
- checking whether the first weight is in a logical relationship with the second weight, wherein there is preferably a logical relationship if the first weight is acquired and the second weight is acquired within one session; and/or
- checking whether the second weight is less than the first weight, and/or checking whether the second weight is greater than 0.5 kg and/or less than 200 kg,
wherein, if the checking was successful, the weight information is marked as complete weight information, and if the checking was unsuccessful, the weight information is marked as incomplete weight information, wherein the checking whether the weight information is complete weight information is carried out before the determination of the patient weight and the patient weight is determined only if the weight information is marked as complete weight information, and/or wherein, if the weight information is marked as incomplete weight information, one, several or all of the steps a)-e) of the method are repeated, preferably at least the steps b) and c) are repeated.

2. Computer-implemented method according to claim 1, wherein, before the second weight is acquired, the unique ID of the patient is acquired again, preferably by reading in a or the patient card.

3. Computer-implemented method according to claim 1 or 2, wherein the first weight is acquired with a first scale and the second weight is acquired with a second scale, wherein the first scale acquires the unique ID before the first weight is acquired, and wherein preferably the second scale acquires the unique ID again before the second weight is acquired.

4. Computer-implemented method according to one of the preceding claims 1 to 3, wherein, if the weight information is marked as incomplete weight information, an indication and/or a warning is output, wherein the indication and/or the warning comprises the first weight, the second weight and/or one, several or all of the information, acquired weights and/or values assigned to the weight information.

5. Computer-implemented method according to one of the preceding claims, wherein, after the determination of the patient weight, an adjuvant weight is determined as the second weight and the adjuvant weight is assigned to the unique ID and/or the weight information, and/or an adjuvant weight assigned to the unique ID and/or the weight information is updated by the second weight.

6. Computer-implemented method according to one of the preceding claims, wherein the method is repeated at least once, such that second weight information is acquired and/or a temporal profile of the first weight, of the second weight and/or of the patient weight is acquired and/or determined, wherein preferably in the case of at least one repetition of the method, instead of the second weight being acquired by weighing, the second weight corresponds to the second weight of a previous implementation of the method, preferably of the immediately previous implementation, and/or is set thereto.

7. System for determining a patient weight of a patient in need of an adjuvant, wherein the system has a computer unit for determining the patient weight and a scale for acquiring a first weight, which corresponds to the weight of the patient and to the weight of the adjuvant, and/or a second weight, which corresponds to the weight of the adjuvant, wherein the scale is preferably further configured to acquire a unique ID of the patient, **characterized in that** the system is configured to carry out the computer-implemented method according to one of the preceding claims 1 to 6.

8. System according to claim 7, wherein the system is further configured to mark the first weight as a post-treatment measurement if
- a treatment, preferably a dialysis treatment, has been completed before acquiring the first weight; and/or
- if a treatment, preferably a dialysis treatment, has been started at a predefined time interval, preferably more than two hours, and has not yet been completed when acquiring the first weight; and/or
- if a pre-treatment measurement is present, preferably a first weight marked as a pre-treatment measurement is present,
wherein the first weight is marked as a pre-treatment measurement if the first weight is not marked as a post-treatment measurement.

9. System according to claim 7 or 8, wherein the system is configured to assign a target weight of the patient, preferably a target weight after a or the treatment, to the unique ID and/or the weight information of the patient, and wherein the checking whether the weight information is a complete weight information comprises:
- checking, if the first weight is marked as a pre-treatment measurement, whether target weight - (first weight - second weight) ≤ ΔG, wherein preferably ΔG ≥ 0, particularly preferably 1 kg ≤ ΔG ≤ 10 kg; and/or
- checking, if the first weight is marked as a pre-treatment measurement, whether (first weight - second weight) - target weight ≤ ΔG, wherein preferably ΔG ≥ 0, particularly preferably 1 kg ≤ ΔG ≤ 10 kg; and/or
- checking, if the first weight is marked as a post-treatment measurement, whether the amount |target weight - (first weight - second weight)| ≤ ΔG, wherein preferably ΔG ≥ 0, particularly preferably 0.1 kg ≤ ΔG ≤ 1 kg,
wherein, if the checking was successful, the weight information is marked as complete weight information, and if the checking was unsuccessful, the weight information is marked as incomplete weight information.

10. System according to one of the preceding claims 8 or 9, in which
- a previous patient weight of the patient determined before the acquisition of the first weight, preferably a previous patient weight of the patient determined before the acquisition of the first weight and after a previous treatment, is present and/or assigned to the unique ID, and wherein the checking whether the weight information is a complete weight information comprises checking, if the first weight is marked as a pre-treatment measurement, whether (first weight - second weight) - previous patient weight ≥ ΔG, wherein preferably ΔG ≥ 0, particularly preferably 1 kg ≤ ΔG ≤ 10 kg; and/or in which
- several previous first weights and previous second weights acquired before the acquisition of the first weight, and/or previously acquired differences (previously acquired first weight - previously acquired second weight) are present, and a reference difference is determined as the difference of the mean deviation of the previously acquired first weights and the mean deviation of the previously acquired second weights, and/or as a mean deviation of the previously acquired differences, and the checking comprises checking whether A ≤ (first weight - second weight) / reference difference ≤ B, wherein preferably A = 1 - x and B = 1 + x with 0 ≤ x ≤ 0.1.

11. System according to one of the preceding claims 7 to 10, which has a second scale, which is configured to acquire the second weight, and preferably the unique ID.

12. System according to one of the preceding claims 7 to 11, in which the computer unit has a database and/or is connected to a database, and the computer unit is configured to store weight information linked to the unique ID of the patient and acquired at different points in time, preferably complete weight information, acquired first weights, acquired second weights, and/or patient weights determined at different points in time in the database, such that a temporal profile can be determined by the computer unit.

13. System according to one of the preceding claims 7 to 12, in which a planned treatment duration of a planned treatment, preferably a planned dialysis duration, and a target weight of the patient, preferably a target weight of the patient after the planned treatment, are known to the computer unit and/or can be retrieved from the computer unit, and wherein the computer unit is configured to determine, if the first weight is a pre-treatment measurement, a planned UF volume from the difference (first weight - second weight) - target weight and further a mean UF rate from the ratio of the planned UF volume to the planned treatment duration.

14. System according to claim 13, in which the computer unit is configured to carry out the determination of the mean UF rate during or after the acquisition of the second weight, and wherein the system is configured to output an indication or a warning if a maximum UF rate characteristic of the patient cannot be maintained, preferably cannot be maintained due to the patient weight.

15. System according to one of the preceding claims 7 to 14, which is configured to release a planned treatment, preferably a planned dialysis treatment, only if the patient weight has been determined and/or if the weight information is a complete weight information.

16. System according to one of the preceding claims 7 to 15, which has a printer, which is configured to print and output a ticket with an indication and/or a warning, one or more weight information, the acquired first weight, the acquired second weight and/or the patient weight.

## Revendications

1. Procédé informatisé pour déterminer le poids d'un patient qui dépend d'un dispositif d'aide, comprenant les étapes suivantes :
a) enregistrement d'un identifiant unique du patient, de préférence par lecture d'une carte de patient ;
b) enregistrement d'un premier poids en pesant le patient doté d'un dispositif d'aide, de préférence un fauteuil roulant, une aide à la marche ou une prothèse, de sorte que le premier poids corresponde à la somme du poids du patient et du poids du dispositif d'aide ;
c) création d'une information de poids liée à l'identifiant unique du patient et attribution du premier poids à l'information de poids ;
d) enregistrement d'un deuxième poids en pesant le dispositif d'aide ;
e) attribution du deuxième poids à l'information de poids ; et
f) calcul du poids du patient en soustrayant le deuxième poids du premier poids, avec de préférence une attribution du poids du patient à l'information de poids et/ou à l'identifiant unique,
**caractérisé en ce que** le procédé comprend la vérification que l'information de poids est une information de poids complète, la vérification comprenant les étapes suivantes :
- vérifier si le deuxième poids a été enregistré ; et/ou
- vérifier si le premier poids a un lien temporel avec le deuxième poids, un lien temporel étant de préférence constitué lorsque l'enregistrement du premier poids et l'enregistrement du deuxième poids ont lieu dans un intervalle de temps prédéfini, de préférence dans les 24 heures ; et/ou
- vérifier si le premier poids a un lien logique avec le deuxième poids, un lien logique étant de préférence constitué lorsque la détection du premier poids et la détection du deuxième poids ont lieu au cours d'une même session ; et/ou
- vérifier si le deuxième poids est inférieur au premier poids, et/ou vérifier si le deuxième poids est supérieur à 0,5 kg et/ou inférieur à 200 kg,
étant entendu que, si la vérification aboutit, l'information de poids est marquée « information de poids complète » et, si la vérification échoue, l'information de poids est marquée « information de poids incomplète », la vérification du caractère complet de l'information de poids étant effectuée avant la détermination du poids du patient, et le poids du patient n'étant calculé que si l'information de poids est marquée « information de poids complète », et/ou étant entendu que, si l'information de poids est marquée comme une information de poids incomplète, une, plusieurs ou toutes les étapes a) à e) du procédé sont répétées, de préférence au moins les étapes b) et c) sont répétées.

2. Procédé informatisé selon la revendication 1, dans lequel l'identifiant unique du patient est à nouveau enregistré avant l'enregistrement du deuxième poids, de préférence par lecture d'une ou de la carte du patient.

3. Procédé informatisé selon la revendication 1 ou 2, dans lequel le premier poids est enregistré à l'aide d'une première balance et le deuxième poids à l'aide d'une deuxième balance, la première balance enregistrant l'identifiant unique avant l'enregistrement du premier poids, et la deuxième balance enregistrant de préférence à nouveau l'identifiant unique avant l'enregistrement du deuxième poids.

4. Procédé informatisé selon l'une des revendications précédentes 1 à 3, dans lequel, lorsque l'information de poids est marquée « information de poids incomplète », une remarque et/ou un avertissement est ou sont émis, la remarque et/ou l'avertissement comprenant le premier poids, le deuxième poids et/ou une, plusieurs ou toutes les informations associées aux informations de poids, aux poids et/ou aux valeurs enregistrés.

5. Procédé informatisé selon l'une des revendications précédentes, dans lequel, après la détermination du poids du patient, un poids auxiliaire est déterminé comme étant le deuxième poids et le poids auxiliaire est associé à l'identifiant unique et/ou à l'information de poids, et/ou un poids auxiliaire associé à l'identifiant unique et/ou à l'information de poids est mis à jour par le deuxième poids.

6. Procédé informatisé selon l'une des revendications précédentes, le procédé étant répété au moins une fois, de sorte qu'une deuxième information de poids est enregistrée et/ou qu'une évolution dans le temps du premier poids, du deuxième poids et/ou du poids du patient est enregistrée et/ou calculée, de préférence, lors d'au moins une répétition du procédé, au lieu d'enregistrer le deuxième poids par pesée, le deuxième poids correspondant au deuxième poids d'une exécution antérieure du procédé, de préférence de l'exécution qui la précède directement, et/ou étant défini par rapport à celle-ci.

7. Système pour calculer le poids d'un patient qui dépend d'un dispositif d'aide, le système présentant une unité informatique pour calculer le poids du patient et une balance pour enregistrer un premier poids correspondant au poids du patient et au poids du dispositif d'aide, et/ou un deuxième poids correspondant au poids du dispositif d'aide, la balance étant de préférence également conçue pour détecter un identifiant unique du patient, **caractérisé en ce que** le système est conçu pour exécuter le procédé informatisé selon l'une des revendications précédentes 1 à 6.

8. Système selon la revendication 7, dans lequel le système est en outre conçu pour marquer le premier poids « mesure post-traitement »
- si un traitement, de préférence un traitement par dialyse, a été réalisé avant la saisie du premier poids ; et/ou
- si un traitement, de préférence un traitement par dialyse, a démarré à un intervalle de temps prédéfini, de préférence supérieur à deux heures, et n'est pas encore terminé lors de l'enregistrement du premier poids ; et/ou
- s'il existe une mesure pré-traitement, de préférence un premier poids marqué « mesure pré-traitement »,
le premier poids étant marqué « mesure pré-traitement » si le premier poids n'est pas marqué « mesure post-traitement ».

9. Système selon la revendication 7 ou 8, dans lequel le système est conçu pour attribuer à l'identifiant unique et/ou à l'information de poids du patient un poids cible du patient, de préférence un poids cible après un ou plusieurs traitements, et dans lequel la vérification du caractère complet de l'information de poids comprend les étapes suivantes :
- si le premier poids est marqué « mesure pré-traitement », vérifier si le poids cible - (premier poids - deuxième poids) ≤ ΔG, où de préférence ΔG ≥ 0, de préférence 1 kg ≤ ΔG ≤ 10 kg ; et/ou
- vérifier, si le premier poids est marqué « mesure pré-traitement », si (premier poids - deuxième poids) - poids cible ≤ ΔG, de préférence ΔG ≥ 0, de préférence 1 kg ≤ ΔG ≤ 10 kg ; et/ou
- vérifier, si le premier poids est marqué « mesure post-traitement », si la valeur | poids cible - (premier poids - deuxième poids) | ≤ ΔG, où de préférence ΔG ≥ 0, de préférence 0,1 kg ≤ ΔG ≤ 1 kg,
étant entendu que, si la vérification aboutit, l'information de poids est marquée « information de poids complète » et, si la vérification échoue, l'information de poids est marquée « information de poids incomplète ».

10. Système selon l'une des revendications 8 ou 9 précédentes, dans lequel
- un poids antérieur du patient déterminé avant la détection du premier poids, de préférence un poids antérieur du patient déterminé avant la détection du premier poids et après un traitement antérieur, est présent et/ou est attribué à l'identifiant unique, et dans lequel la vérification que l'information de poids est une information de poids complète comprend une vérification, lorsque le premier poids est marqué « mesure pré-traitement », du fait que (premier poids - deuxième poids) - poids antérieur du patient ≥ ΔG, où de préférence ΔG ≥ 0, de préférence 1 kg ≤ ΔG ≤ 10 kg ; et/ou dans lequel
- plusieurs premiers poids antérieurs et deuxièmes poids antérieurs enregistrés avant l'enregistrement du premier poids, et/ou des différences antérieurement enregistrées (premier poids antérieurement enregistré - deuxième poids antérieurement enregistré) sont disponibles, et une différence de référence est ou sera déterminée comme étant la différence entre l'écart moyen des premiers poids antérieurement enregistrés et l'écart moyen des deuxièmes poids antérieurement enregistrés, et/ou comme étant un écart moyen des écarts antérieurement enregistrés, et la vérification consistant à vérifier si A ≤ (premier poids - deuxième poids) / différence de référence ≤ B, de préférence A = 1 - x et B = 1 + x avec 0 ≤ x ≤ 0,1.

11. Système selon l'une des revendications 7 à 10 précédentes, qui présente une deuxième balance qui est conçue pour enregistrer le deuxième poids et, de préférence, l'identifiant unique.

12. Système selon l'une des revendications 7 à 11 précédentes, dans lequel l'unité informatique présente une base de données et/ou est connectée à une base de données, et l'unité informatique est conçue pour enregistrer des informations de poids associées à l'identifiant unique du patient et enregistrées à différents moments, de préférence des informations de poids complètes, des premiers poids enregistrés, des seconds poids enregistrés, et/ou les poids du patient déterminés à différents moments, de sorte que l'unité informatique puisse déterminer une évolution dans le temps.

13. Système selon l'une des revendications 7 à 12 précédentes, dans lequel la durée prévue d'un traitement prévu, de préférence la durée prévue d'une dialyse, et un poids cible du patient, de préférence un poids cible du patient après le traitement prévu, sont connus de l'unité informatique et/ou peuvent être consultés par l'unité informatique, et dans lequel l'unité informatique est conçue pour déterminer, lorsque le premier poids est une mesure pré-traitement, un volume d'UF prévu à partir de la différence (premier poids - deuxième poids) - poids cible et, en outre, un débit moyen d'UF à partir du rapport entre le volume d'UF prévu et la durée de traitement prévue.

14. Système selon la revendication 13, dans lequel l'unité informatique est conçue pour procéder au calcul du taux moyen d'UF lors de la saisie du deuxième poids ou après celle-ci, et dans lequel le système est conçu pour émettre une indication ou un avertissement lorsqu'un taux maximal d'UF caractéristique du patient ne peut être respecté, de préférence en raison du poids du patient.

15. Système selon l'une des revendications 7 à 14 précédentes, qui est conçu pour n'autoriser un traitement prévu, de préférence un traitement de dialyse prévu, que lorsque le poids du patient a été calculé et/ou lorsque l'information de poids est complète.

16. Système selon l'une des revendications 7 à 15 précédentes, qui présente une imprimante qui est conçue pour imprimer et délivrer un ticket avec une remarque et/ou un avertissement, une ou plusieurs informations de poids, le premier poids enregistré, le deuxième poids enregistré et/ou le poids du patient.
